# EUROPÄISCHE PATENTSCHRIFT

(11) **EP 1 430 304 B1**
(45) Veröffentlichungstag und Bekanntmachung des Hinweises auf die Patenterteilung: **06.12.2006**
(21) Anmeldenummer: 02774558.7
(22) Anmeldetag: 03.09.2002
(51) Int. Cl.: G01N 33/564

(54) **VERFAHREN ZUR DIAGNOSE VON CHRONISCH ENTZÜNDLICHEN DARMERKRANKUNGEN**
METHOD FOR DIAGNOSING CHRONIC INFLAMMATORY BOWEL DISEASES
PROCEDE POUR LE DIAGNOSTIC DE MALADIES INFLAMMATOIRES CHRONIQUES INTESTINALES

(30) Priorität: 28.09.2001 DE 10147991
(43) Veröffentlichungstag der Anmeldung: 23.06.2004
(73) Patentinhaber: B.R.A.H.M.S Aktiengesellschaft, 16761 Hennigsdorf (DE)
(72) Erfinder: BERGMANN, Andreas, 12351 Berlin (DE); WILLNICH, Marita, 10247 Berlin (DE); SCHUPPAN, Detlef, 91088 Bubenreuth (DE)
(74) Vertreter: Andrae, Steffen
(86) Internationale Anmeldenummer: PCT/EP2002/009848
(87) Internationale Veröffentlichungsnummer: WO 2003/029824

(56) Entgegenhaltungen:
- US-A- 4 865 970
- FABIEN NICOLE ET AL: "Autoantibodies directed against the ribosomal P proteins are not only directed against a common epitope of the P0, P1 and P2 proteins." JOURNAL OF AUTOIMMUNITY, Bd. 13, Nr. 1, August 1999 (1999-08), Seiten 103-110, XP002232563 ISSN: 0896-8411 in der Anmeldung erwähnt
- FRIGERIO JEAN-MARC ET AL: "Cloning, sequencing and expression of the L5, L21, L27a, L28, S5, S9, S10 and S29 human ribosomal protein mRNAs." BIOCHIMICA ET BIOPHYSICA ACTA, Bd. 1262, Nr. 1, 1995, Seiten 64-68, XP002915514 ISSN: 0006-3002 in der Anmeldung erwähnt
- BARNARD G F ET AL: "INCREASED EXPRESSION OF HUMAN RIBOSOMAL PHOSPHOPROTEIN P0 MESSENGER RNA IN HEPATOCELLULAR CARCINOMA AND COLON CARCINOMA" CANCER RESEARCH, Bd. 52, Nr. 11, 1992, Seiten 3067-3072, XP001145714 ISSN: 0008-5472 in der Anmeldung erwähnt
- ROOZENDAAL C ET AL: "Are anti-neutrophil cytoplasmic antibodies (ANCA) clinically useful in inflammatory bowel disease (IBD)?" CLINICAL AND EXPERIMENTAL IMMUNOLOGY, Bd. 116, Nr. 2, Mai 1999 (1999-05), Seiten 206-213, XP002232564 ISSN: 0009-9104

## Beschreibung

Die vorliegende Erfindung betrifft neue Verfahren zur Diagnose entzündlicher Darmerkrankungen, insbesondere zur Diagnose der chronisch entzündlichen Darmerkrankungen Morbus Crohn (MC) und Colitis ulcerosa (CU) und ihrer Mischform, die als Colitis indeterminata bezeichnet wird, die auf dem erstmaligen Nachweis definierter Antikörper (Autoantikörper) bei Patienten, die an derartigen Erkrankungen leiden, beruhen, sowie daraus ableitbare Verwendungen der zugehörigen Antigene (Proteine).

Zu den Erkrankungen des Gastrointestinaltrakts gehören auch entzündliche Darmerkrankungen, bei denen die Darmwand und/oder Schleimhaut des Darms entzündet ist. Als Enteritis im engeren Sinn wird eine Entzündung der Darmwand des Dünndarms bezeichnet, während man als Colitis Schleimhautentzündungen des Dickdarms bezeichnet. Entzündliche Darmerkrankungen können verschiedene Ursachen haben, beispielsweise infektiöser Natur sein oder in der Folge von Vergiftungen auftreten, und sie können als akute oder chronische Erkrankungen auftreten. Unter den entzündlichen Darmerkrankungen sind chronisch entzündliche Darmerkrankungen aufgrund der hohen assoziierten Morbidität (Erkrankungsrate) und Mortalität (Sterberate) von besonderer Bedeutung für die Volkswirtschaft und die innere Medizin: die unter den Bezeichnungen Morbus Crohn (Synonyma: Enteritis regionalis, Crohn Krankheit) und Colitis ulcerosa (Synonyma: Colitis gravis) bekannten Erkrankungen sowie ihre Mischform Colitis indeterminata. Charakteristische Symptome sind abdominelle Schmerzen, Diarrhöen, und bei Dünndarmbefall ggf. Malabsorption. Als Komplikationen treten beim Morbus Crohn Darmstenosen, innere und äußere Fisteln und Abszessbildungen auf, bei der Colitis ulcerosa dagegen zum Teil schwere Blutverluste und ein erhöhtes Risiko des Auftretens von Dickdarmkarzinomen (ca. 40% bei 25-jährigem Krankheitsverlauf). Klinische Charakteristika sind beim Morbus Crohn der diskontinuierliche Befall des distalen Dünndarms und des Colons, während bei der Colitis ulcerosa ein kontinuierlicher Darmbefall, primär der distalen Abschnitte, vorliegt. Histologisch ist für Morbus Crohn der Befund von Epitheloidzellgranulomen charakteristisch, und für Colitus ulcerosa der Befund von Kryptenabszessen. 5-10% der an chronisch entzündlichen Darmerkrankungen leidenden Patienten zeigen Charakteristika beider Krankheitsbilder (Colitis indeterminata).

Nach dem bisherigen Wissensstand sind die chronisch entzündlichen Darmerkrankungen multifaktoriell bedingt. Zu den diskutierten Faktoren gehören Ernährungsgewohnheiten und psychische Auslöser. Eine polygenetische Prädisposition und eine Reihe von externen Einflüssen, am ehesten aus der Darmflora, gelten als gesichert.

Aufgrund des chronischen Krankheitsverlaufs und verschiedener Begleitphänomene werden die genannten chronisch entzündlichen Darmerkrankungen seit längerem auch unter dem Gesichtspunkt möglicher Autoimmunreaktionen diskutiert. Es sind bis heute jedoch weder Autoantikörper charakterisiert worden, deren Auftreten die für diagnostische Zwecke benötigte Sensitivität (auf Erkrankte bezogener Kennwert; bei einer optimalen Sensitivität von 100% werden die Antikörper bei allen Erkrankten gefunden) und Spezifität (auf Gesunde bezogener Kennwert; bei einer optimalen Spezifität von 100% sind die Antikörper in keinem Falle bei Gesunden nachweisbar) aufweist. Die mangelnde Charakterisierung von eng an das Krankheitsgeschehen der chronisch entzündlichen Darmerkrankungen gekoppelten Antikörper hat es bisher auch verhindert, bei der Therapie derartiger Erkrankungen ihren Charakter als mögliche Autoimmunerkrankungen in irgendeiner Weise gezielt zu berücksichtigen.

Die vorliegende Erfindung beruht auf neuen Erkenntnissen, die mit einem speziell konzipierten analytischen Verfahren der immunologischen Proteinanalyse gewonnen wurden, das rein empirisch ist und in das außer der Annahme, dass eine sich in einem speziellen Organ bzw. Gewebe manifestierende zu untersuchende Erkrankung eine Autoimmunerkrankung sein könnte, keine zusätzlichen hypothetischen oder theoretischen Ansätze eingehen. Das angewandte Analyseverfahren wird im folgenden noch genauer erläutert.

Die Anwendung des Verfahrens führte zu dem Ergebnis, dass bei Patienten, bei denen aufgrund der klinischen Befunde Morbus Crohn bzw. Colitis ulcerosa diagnostiziert worden war, mit hoher Sensitivität und Spezifität Antikörper gefunden werden, die an definierte ribosomale Proteine binden, die sich als solche als bekannt erwiesen haben, nämlich die ribosomalen Proteine P0 und/oder L5. Deren Beteiligung an einem Autoimmungeschehen, das bei Morbus Crohn bzw. Colitis ulcerosa eine Rolle spielt, war bisher völlig unbekannt.

Die hierin beschriebenen experimentiellen Befunde machen es erstmals möglich, die genannten chronisch entzündlichen Darmerkrankungen durch einen entsprechenden Antikörpernachweis in biologischen Proben, insbesondere in Serum, Plasma, Gewebeproben und/oder auch im Stuhl, mit hoher diagnostischer Aussagekraft zu diagnostizieren.

Die neuartigen Erkenntnisse über die Natur der bei den genannten Erkrankungen auftretenden Antikörper macht es ferner möglich, neuartige therapeutische Ansätze zur Behandlung der genannten Erkrankungen zu entwickeln, die an deren Charakter als Autoimmunerkrankungen ansetzen.

Gemäß der vorliegenden Erfindung wird somit unter einem ersten Aspekt ein neuartiges diagnostisches Verfahren, und zwar zur Diagnose, zur Frühdiagnose, zur Differentialdiagnose, zur Beurteilung der Schwere und zur therapiebegleitenden Verlaufskontrolle und Verlaufsprognose, von chronisch entzündlichen Darmerkrankungen, insbesondere Morbus Crohn, Colitis ulcerosa und ihrer Mischform Colitis indeterminata, geschaffen, das dadurch gekennzeichnet ist, dass man im Serum, Plasma, Gewebeproben und/oder im Stuhl von Patienten, die an einer entzündlichen Darmerkrankung leiden, oder bei denen der Verdacht auf eine derartige Erkrankung besteht, das Vorhandensein und/oder die Menge von einem oder mehreren Antikörpern bestimmt, die an ribosomale Proteine, insbesondere die ribosomalen Proteine P0 oder L5, binden.

Die Verfahren zur Bestimmung der genannten Antikörper in einer biologischen Probe können beliebige bekannte Verfahren der Immundiagnostik sein, die zum Nachweis und zur Messung von Antikörpern angewandt werden. Vorzugsweise werden die Antikörper mit Hilfe eines Immunoassays bestimmt, bei dem man als Antigen zur Bindung der gesuchten Antikörper das jeweilige ribosomale Protein (P0, L5) verwendet. Zur Markierung der aus einer biologischen Probe spezifisch gebundenen Antikörper kann man dann auf irgendeine geeignete, an sich bekannte Weise markierte Anti-Human-Antikörper verwenden, oder eine weitere Antigenpräparation, die das zur Antikörperbindung benutzte Antigen, oder ein ähnliches spezifisches Antigen, in markierter Form enthält. Selbstverständlich ist vorzugsweise ein solcher Assay zur Antikörperbestimmung zu verwenden, der die benötigte Empfindlichkeit im Bereich der vorkommenden Antikörper-Konzentrationen gewährleistet.

Das Bestimmungsverfahren kann auch in der Chip-Technologie oder als Schnelltest (Point-of-Care-Test) ausgeführt werden. Das als Antigen zu verwendende ribosomale Protein P0 und/oder L5 kann dabei ein humanes oder tierisches Protein sein, das aus geeigneten natürlichen (humanen oder tierischen) Quellen angereichert oder isoliert wurde, oder das man gezielt auf gentechnologischem Wege (rekombinant) erzeugt hat. Da z.B. das ribosomale Protein P0 über viele Artgrenzen hinweg in identischer oder immunologisch sehr ähnlicher Form gefunden wird, steht für die Schaffung von geeigneten Assays eine breite Palette von möglicherweise geeigneten Ausgangsmaterialien zur Verfügung, aus denen die benötigten Antigenpräparationen gewonnen werden können.

Zur Schaffung neuartiger Therapiemöglichkeiten der chronisch entzündlichen Darmerkrankungen Morbus Crohn und Colitis ulcerosa können therapeutische Ansätze verfolgt werden, die einerseits darauf abzielen, die am Autoimmungeschehen beteiligten Antikörper zu desaktivieren (blockieren) oder zu entfernen, oder andererseits das Krankheitsgeschehen gezielt durch die Erzeugung einer Immuntoleranz zu beeinflussen. Zur Blockierung der Antikörper können Wirksubstanzen, beispielsweise Ribosomen, Ribosomenfraktionen, ribosomale Proteine oder deren Fragmente oder Derivate verwendet werden, die die zirkulierenden Antikörper binden und deaktivieren. Derartige spezifische Binder für die genannten Antikörper können auch zur Herstellung von Materialien für die Affinitätsreinigung verwendet werden, mittels derer im Sinne einer Plasmapherese die pathogenen Antikörper extrakorporal entfernt werden können.

Alternativ können die Proteine P0 und/oder L5 verwendet werden, um in antigenpräsentierenden Zellen oder T-Zellen durch Blockierung oder Modulation der Antigenpräsentation eine Immuntoleranz oder eine Blockade der T-Zell-Reaktivität zu induzieren.

Die oben summarisch dargestellten Ergebnisse wurden mittels der folgenden, allgemein anwendbaren analytischen Vorgehensweise erhalten:

Zur Identifizierung von Antikörpern (Autoantikörpern) wurde ein rein empirisches Analyseverfahren entwickelt, das geeignet ist, eine Beteiligung eines Autoimmungeschehens an prinzipiell beliebigen Erkrankungen festzustellen, und das gleichzeitig genaue Informationen über die Bindungspartner der bei der jeweiligen Erkrankung auftretenden Autoantikörper liefert.

Es wird wie folgt vorgegangen:
Wird vermutet, dass eine Erkrankung auf Autoimmunreaktionen beruht oder eine Autoimmunkomponente aufweist, wird unterstellt, dass die für Autoimmunerkrankungen charakteristischen Autoantikörper Gewebestrukturen derjenigen Organe oder Gewebe angreifen, bei denen die für die jeweiligen Erkrankungen typischen Krankheitserscheinungen besonders deutlich ausgeprägt sind. Zur Prüfung der Frage, ob am Krankheitsgeschehen typische Autoantikörper beteiligt sind, wird dann zuerst durch eine unspezifische Affinitätsreinigung eine Immunglobulinfraktion von Patienten gewonnen, die aufgrund der klinischen Befunde an der zu untersuchenden Erkrankung leiden. Parallel dazu wird eine entsprechende Immunglobulinfraktion von Gesunden gewonnen. Die erhaltenen Immunglobulinfraktionen von Gesunden und Erkrankten werden dann getrennt an Trägermaterialien für eine Affinitätschromatographie gebunden, so dass zwei durch unterschiedliche Immunglobuline charakterisierte Säulen für eine vergleichende Affinitätschromatographie erhalten werden.

Beide Säulen werden dann mit einem Gewebeextrakt beschickt, der aus einem gesunden Gewebe, jedoch auch aus einem pathologischen Gewebe gewonnen werden kann, das im Falle der Erkrankung angegriffen wird.

Wenn ein-und-derselbe Gewebeextrakt über die beiden unterschiedlichen Affinitätssäulen geleitet wird, werden dann, wenn an die mit Patienten-Immunglobulinen beladene Affinitätssäule krankheitstypische zusätzliche Autoantikörper gebunden sind, aus dem aufgegebenen Gewebeextrakt solche Komponenten zurückgehalten, die mit den vorhandenen Autoantikörpern auf der Säule eine spezifische Bindungsreaktion eingehen.

Bei der nachfolgenden Elution der Säulen unter Bedingungen, unter denen Antigen-Antikörper-Bindungen gelöst werden, werden zwei verschiedene Eluate (Proteinfraktionen) erhalten, wobei das Eluat aus der Affinitätssäule mit den Patienten-Immunglobulinen ggf. zusätzliche Bestandteile enthält, die an vorhandene krankheitsspezifische Autoantikörper gebunden waren.

Untersucht man die Zusammensetzung der Eluate anschließend durch 2D-Gelelektrophorese, werden die vorher von den krankheitsspezifischen Antikörpern gebundenen Komponenten der Gewebeprobe als zusätzliche Spots von Proteinen erkennbar, die von der autoantikörperfreien Affinitätssäule mit Immunglobulinen von Gesunden nicht zurückgehalten wurden.

Die krankheitstypischen Proteinspots können dann isoliert und mittels moderner Verfahren der Proteinanalyse untersucht werden.

Setzt man eine Gewebefraktion aus einem gesunden Gewebe ein, sind die gefundenen zusätzlichen Proteine nicht notwendigerweise die Autoantigene, die an der Entstehung der Autoimmunreaktion ursächlich beteiligt waren. Wird beispielsweise die Autoimmunreaktion durch besondere pathologische Formen der gefundenen Proteine (z.B. speziell prozessierte Proteine, organspezifische Splicing-Varianten, Fremdproteine, die z.B. auf infektiösem Weg in den Darm gelangten) ausgelöst/bedingt, muss sich das im Bindungsverhalten der isolierten Antikörper gegen die im Test verwendeten "normalen" Gewebsbestandteile nicht niederschlagen. Die genaue Natur der pathogen wirkenden (auslösenden) Autoantigene ist für die Immundiagnostik, bei der es nur um die sichere Zuordnung von Biomarkern zu einem Krankheitsgeschehen geht, nur von sekundärer Bedeutung. Selbstverständlich ist es jedoch im Lichte der Erkenntnisse über das krankheitsspezifische Auftreten bestimmter Autoantikörper grundsätzlich auch möglich, unter Einsatz von Gewebeproben Erkrankter, ggf. in Verbindung mit Antikörpern der gefundenen Art, genauere Details über die tatsächlich in den angegriffenen Geweben vorhandenen funktionellen Veränderungen zu ermitteln, die krankheitsauslösend sind oder bei den Erkrankten mit den Autoantikörpern in Wechselwirkung treten. Derartige Folgeerkenntnisse können dann ggf. zu einer weiteren Verbesserung diagnostischer Verfahren genutzt werden.

Die Versuche der Erfinder wurden im vorliegenden Falle, in dem es um die Gewinnung von Erkenntnissen zu chronisch entzündlichen Darmerkrankungen ging, mit aus dem Dünndarm von (gesunden) Pavianen gewonnenen Gewebeextrakten durchgeführt. Der Gewebeextrakt aus Primaten (Pavianen) wurde wegen seiner leichteren Verfügbarkeit sowie aufgrund der erfahrungsgemäß sehr hohen Ähnlichkeit der Proteine von Primaten und Menschen gewählt, die sich als hohe Kreuzreaktivitäten mit vielen therapeutischen und diagnostischen humanen Reagenzien äußert.

Indem man, wie nachfolgend näher beschrieben, mit dem Gewebeextrakt aus dem Dünndarm von Pavianen in Kombination mit Affinitätssäulen arbeitete, von denen eine mit einer affinitätsgereinigten Immunglobulinfraktion von Gesunden belegt war, während die andere eine Immunglobulinfraktion von Morbus Crohn- und/oder Colitis ulcerosa-Patienten aufwies, konnten in den Eluaten aus den Patienten-Affinitätssäulen zwei Proteinspots gefunden werden, die nur in den Eluaten aus den Affinitätssäulen mit den Patienten-Immunglobulinen auftraten, und die nach Maßgabe der Gelektrophorese Molekulargewichte von etwa 36 kD bzw. 38 kD aufwiesen. Die Proteinspots wurden aus dem Elektrophoresegel isoliert und durch Trypsin-Verdauung in Bruchstücke zerlegt, die auf an sich bekannte Weise massenspektrometrisch analysiert wurden und durch Vergleich mit den Daten für bekannte Trypsinbehandelte Proteine identifiziert werden konnten.

Es zeigte sich, dass beide gefundenen Proteine bekannte ribosomale Proteine waren, und zwar einerseits das sog. ribosomale Protein P0, das die bekannte Sequenz gemäß SEQ ID NO:2 (Datenbank SWISS PROT Entry RLA0_HUMAN 60S acidic ribosomal protein P0; P05388; vgl. auch N. Fabien et al., (15), Autoantibodies Directed Against the Ribosomal P Proteins are not only Directed Against a Common Epitope of the P0, P1 and P2 Proteins, J.Autoimmun. (1999) 13, 103-110; und die darin zitierte Literatur, insbesondere Wool I.G. et al., Biochimie 73:861-870;) aufweist, sowie andererseits das ribosomale Protein L5, das die bekannte Sequenz gemäß SEQ ID NO:1 aufweist (Datenbank SWISS PROT Entry RL5_HUMAN 60S ribosomal protein L5; P46777; vgl. auch J.-M. Frigerio, J.-C. Dagorn, J.L. Iovanna, (26), Cloning, sequencing and expression of the L5, L21, L27a, L28, S5, S9, S10 and S29 human ribosomal protein mRNAs, Biochim.Biophys.Acta 1262 (1995) 64-68).

Es war zwar bekannt, dass beide Proteine bzw. gegen diese gerichtete Antikörper im Rahmen von Autoimmunerkrankungen, und zwar beim sog. Systemischen Lupus erythematodes (SLE), eine Rolle spielen (vgl. z.B. J.-C. Homberg, M. Rizzetto and Deborah Doniach, (2), Ribosomal Antibodies Detected by Immunofluorescence in Systemic Lupus Erythematosus and other Collagenoses, Clin.exp.Immunol. (1974) 17, 617-628; Thomas L., (1), Labor und Diagnose, 5. Auflage, 1998, 824-842; A.Giualis et al., (22), Anti-5S RNA/protein (RNP) antibody levels correlate with disease activity in a patient with systemic lupus erythematosus (SLE) nephritis, Clin.Exp.Immunol. 1994, 95, 385-389). Antikörper gegen das Protein P0 werden im Rahmen der Diagnose von SLE klinisch bestimmt. Ein für diesen Zweck entwickelter Assay ist beschrieben in US-A-4,865,970. Ein ähnlicher Assay wird ferner vertrieben als ImmuLisa^{™} Anti-Ribosomal P Kit, ELISA, IMMCO Diagnostics Inc., Buffalo, NY 14228. Diese Assays arbeiten mit einer relativ kurzen Peptidsequenz vom Carboxy-Terminus des Proteins P0 und der nahe verwandten Proteine P1 und P2 als spezifischer Binder für die zu bestimmenden Antikörper. Ein solcher Assay kann auch im Rahmen des erfindungsgemäßen Diagnose-Verfahren verwendet werden, ist jedoch weniger bevorzugt, da mit Teilpeptiden der genannten Art nur ein Teil der relevanten Antikörper gefunden wird (N. Fabien et al., (22), J.Autoimmun. (1999) 13, 103-110).

Dass auch bei den chronisch entzündlichen Darmerkrankungen Morbus Crohn (MC) und Colitis ulcerosa (CU) in Patientenseren Autoantikörper gefunden werden, die an die genannten beiden ribosomalen Proteine P0 und L5 binden, war bisher völlig unbekannt.

P0 ist ein ribosomales Protein, das sich in identischer Form oder in Form von ribosomalen Proteinen mit einer sehr hohen Homologie in zahlreichen Organismen findet, die von niederen Organismen, z.B. Parasiten, bis zum Menschen reichen. Es ist bekannt, dass es in Pentamer-Komplexen in der großen Untereinheit von Ribosomen auftritt. Zu dem ribosomalen Protein P0 existiert eine umfangreiche wissenschaftliche Literatur, auf die hiermit pauschal unter Verweis auf die Beispiele in der beigefügten Literaturliste verwiesen wird, insbesondere die Nummern 1-16. Im Hinblick auf die Befunde in der vorliegenden Anmeldung ist dabei von besonderem Interesse, dass P0 bei Darm- und Lebercarcinomen überexprimiert (7,8) wird, und je nach Gewebe auch auf der Oberfläche von Zellmembranen exprimiert wird (5), was im Hinblick auf die Darmspezifität von MC und CU von Interesse ist. Es ist ferner zu erwähnen, dass Antikörper gegen ribosomale P-Proteine, zu den P0 gehört, auch unter dem Gesichtspunkt von Anti-Lymphocyten-Antikörpern diskutiert werden (3,4).

Auch zu dem ribosomalen Protein L5 existiert eine umfangreiche wissenschaftliche Literatur. Es wird in diesem Zusammenhang wiederum pauschal auf die Beispiele in der Literaturliste, insbesondere Nummern 18-27, verwiesen. Im Zusammenhang mit den Befunden in der vorliegenden Anmeldung ist von besonderem Interesse, dass L5 mit zahlreichen Proteinen, z.B. solchen mit Enzymfunktion (z.B. Proteinphosphatase-1 (19) oder Calmodulin-Kinase-2 (27)), wechselwirkt, und dass L5 ferner in Komplexen mit RNA (19-23) auftritt und in dieser komplexierten Form als Antigen wirken kann. Es ist darüberhinaus zu erwarten, dass die Befunde in der vorliegenden Anmeldung der Forschung neuen starke Impulse verleihen werden.

Nachfolgend wird die Auffindung und Identifizierung der Proteine, die nur an bei den MC- bzw. CU-Patienten nachweisbare Antikörper banden, in näheren Einzelheiten geschildert, wobei auf die beigefügten Sequenzprotokolle bezug genommen wird.

Die Figuren zeigen:
- Fig. 1: Ansichten von 2D-Elektrophoresegelen, die einen Vergleich der Spotmuster der Eluate einer Affinitätssäule mit Immunglobulinen aus Seren von Gesunden (A) mit denen einer Affinitätssäule mit den Immunglobulinen aus Seren von MC- bzw. CU-Patienten (B) ermöglichen. Der umrandete Bereich zeigt die Position des ersten nur aus den PatientenAffinitätssäulen eluierbaren Proteins an;
- Fig. 2: Ansichten von 2D-Elektrophoresegelen, die einen Vergleich der Spotmuster der Eluate einer Affinitätssäule mit Immunglobulinen aus Seren von Gesunden (A) mit denen einer Affinitätssäule mit den Immunglobulinen aus Seren von MC- bzw. CU-Patienten (B) ermöglichen. Der umrandete Berich zeigt die Position des zweiten nur aus den Patienten-Affinitätssäulen eluierbaren Proteins an;
- Fig. 3: das Massenspektrum des aus dem Gel der 2D-Gelelektrophorese gemäß Fig.1(B) isolierten, trypsinverdauten Produkts.
- Fig. 4: das Massenspektrum des aus dem Gel der 2D-Gelelektrophorese gemäß Fig.2(B) isolierten, trypsinverdauten Produkts.

### 1. Herstellung eines Extrakts aus dem Dünndarm von Pavianen

Erwachsene Paviane (2-3 Jahre alt, 25-35 kg, männlich und weiblich) wurden mittels i.V. Gabe von Dolethal (10 ml) getötet. Der Dünndarm wurde innerhalb von 15 min entnommen, mit Wasser gewaschen, in Stücke von ca. 10 g zerteilt und unmittelbar in flüssigem Stickstoff eingefroren.

Bei der weiteren Verarbeitung wurden Proben der einzelnen tiefgefrorenen Dünndarmgewebe unter Stickstoffkühlung in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Alle weiteren Schritte erfolgten bei +4°C. Das Pulver wurde in 100 ml Puffer (50 mM Hepes, 50 mM NaCl, pH 8) aufgenommen und mittels eines 60-ml-Potters (Fa. Braun Melsungen) in 5 Auf-und-Ab-Bewegungen bei 900 U/min homogenisiert. Nach einer anschließenden 1-stündigen Zentrifugation bei 100.000 g wurde der erhaltene Überstand (Gewebsextrakt) gewonnen und für die weiteren Untersuchungen verwendet.

### 2. Herstellung von Immunglobulin-Präparationen und Affinitätssäulen

Aus Seren von Patienten mit Morbus Crohn bzw. Colitis ulcerosa einerseits und von Gesunden andererseits (Kontrollseren) wurden mittels einer unspezifischen Affinitätsreinigung über Protein-G-Agarose die Immunglobuline isoliert. Zu diesem Zwecke wurden je 0,2 ml Serum mit 0,2 ml PBS gemischt und anschließend mit 0,5 ml Protein-G-Agarose (gepacktes Gel) versetzt. Die Mischung wird 30 min unter leichtem Schütteln inkubiert, und anschließend in eine Glassäule (Durchmesser 0,5 cm), die am unteren Ende mit einer Feinfritte verschlossen war, gefüllt. Die Säule wird zuerst mit PBS (5 ml) gewaschen, dann werden gebundene Immunglobuline mit 20 mM Citronensäure (pH ca. 2,5) eluiert (Flussgeschwindigkeit ca. 1 ml/min). Das Immunglobulin-Eluat wird durch Zusatz von TRIS-HCl, 1 M, pH 8,0 neutralisiert.

Die gereinigte Immunglobulin-Fraktion wird anschließend unter Zusatz von Natrumperiodat (Endkonzentration 10 mg/ml) 20 min oxidiert. Das Natriumperiodat wird danach durch Entsalzen mittels einer NAP-5-Säule (Pharmacia) nach der Vorschrift des Herstellers entfernt, wobei in der Säule und als Elutionspuffer PBS verwendet wird.

Die entsalzte oxidierte Immunglobulin-Fraktion wird mit Carbolink-Material (0,5 ml gepacktes Gel, gewaschen mit PBS, Fa. Pierce) vermengt. Nach einer 12 h Inkubation unter leichtem Schütteln wird das erhaltene, mit den jeweiligen Immunglobulin-Fraktionen belegte Affinitätsmaterial in eine Glassäule (Durchmesser 0,5 cm) mit Feinfritte gefüllt und mit 10 ml PBS gewaschen.

### 3. Aufarbeitung des Darmextrakts durch Affinitätsreinigung

Je 5 ml eines Pavian-Darmextrakts gemäß 1. werden bei einer Fliessgeschwindigkeit von 0,5 ml/min für eine Stunde kontinuierlich wiederholt über jeweils eine mit einer Immunglobulin-Fraktion gemäß 2. belegte Affinitätssäule gegeben. Anschließend werden die Säulen mit 5 ml PBS gewaschen. Der Ausfluss wird kontinuierlich auf Absorption bei 280 nm überwacht.

Die an die Affinitätssäulen gebundenen Proteine werden danach mit 20 mM Citronensäure (pH ca. 2,5) eluiert. Die dabei erhaltenen Eluate werden anschließend mittel 2-D-Gelektrophorese analysiert.

### 4. Proteomanalyse unter Verwendung der Eluate der Affinitätssäulen.

Bei der einleitenden analytischen 2D-Gelelektrophorese wurden die einzelnen Eluate mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32). Die angefärbten Gele wurden mit einem Biorad 583 Geltrockner getrocknet.

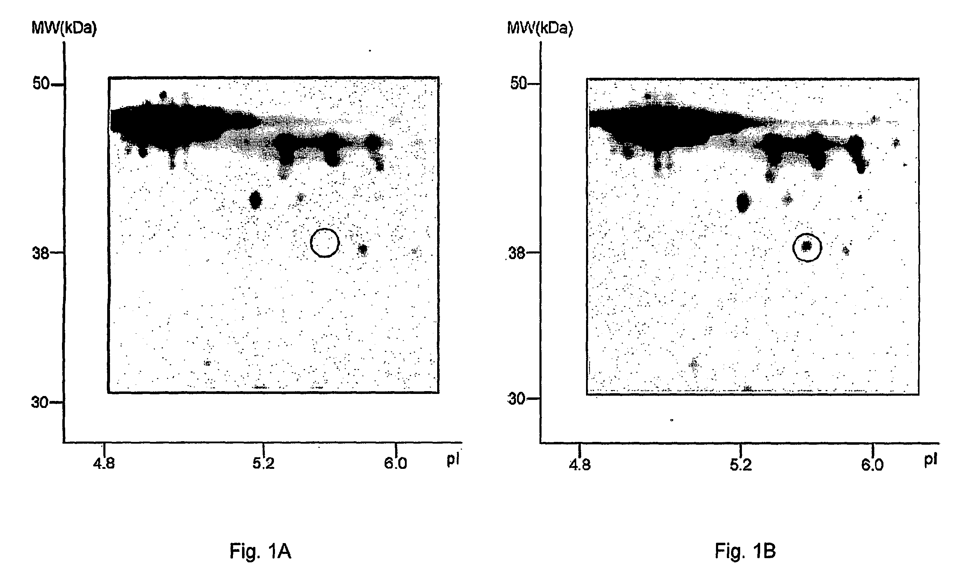

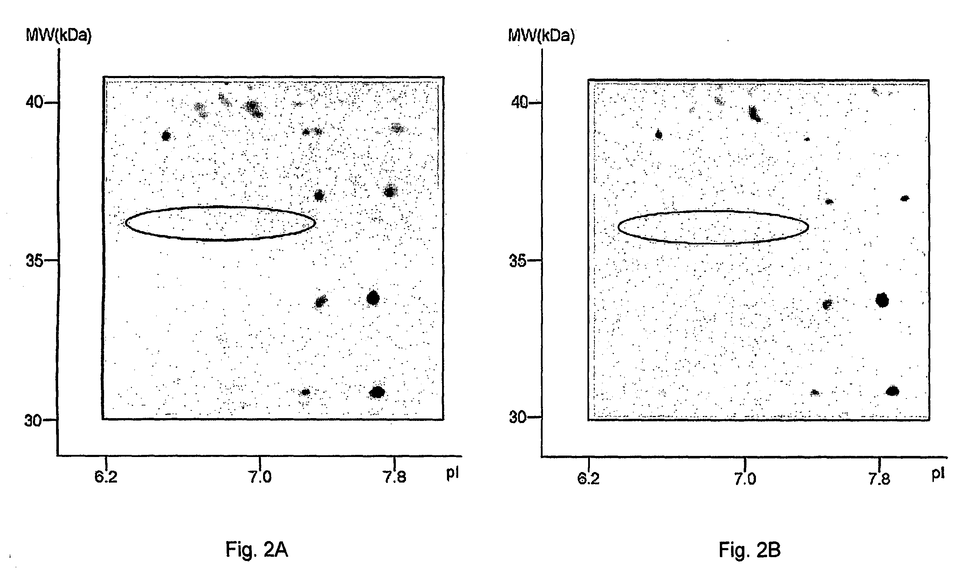

Zur Auswertung wurden das Proteinspotmuster eines Eluats aus der Affinitätssäule mit den Immunglobulinen Gesunder mit dem Proteinspotmuster verglichen, das aus einem entsprechenden Eluat aus der Affinitätssäule mit Immunglobulinen von MC- bzw. CU-Patienten resultierte. Proteinspots, die nie bei Gesunden, jedoch stets bei Patientenproben zusätzlich auftraten, wurden für weitere analytische Untersuchungen selektiert. Fig. 1 und Fig.2 zeigen Aufnahmen von 2D-Elektrophoresegelen, die einen Vergleich der Spotmuster der Eluate einer Affinitätssäule mit Immunglobulinen aus Seren von Gesunden (A) mit denen einer Affinitätssäule mit den Immunglobulinen aus Seren von MC- bzw. CU-Patienten (B) ermöglichen. Die umrandeten Bereiche zeigen die Positionen der nur aus den Patienten-Affinitätssäulen eluierbaren Proteine an.

Überraschend wurden in den Eluaten aus der Patientensäule zwei neue Proteinspots bzw. -banden gefunden: Ein Spot bei einem Molekülgewicht von ca. 38 kD und einem pI von ca. 5,5, und eine scharfe Bande beim Molekülgewicht von ca. 36 kD, die allerdings in pI-Richtung als langezogener streifenartiger Spot erschien. Die beiden neuen Proteinspots traten bei allen Eluaten aus Patientensäulen auf, waren jedoch in den Eluaten aus den Säulen mit den Immunglobulinen Gesunder nicht sichtbar.

Die im Proteinspotmuster der analytischen 2D-Gelelektrophorese identifizierten neuen spezifischen Proteine wurden dann anschließend mittels präparativer 2D-Gelelektrophorese präpariert (vgl. Klose, a.a.0.). Die Färbung erfolgte mittels Coomassie Brilliant Blue G250 (vgl. V.Neuhoff, et al., Electrophoresis 1988, 9, 255-262; sowie Electrophoresis 1990, 11, 101-117).

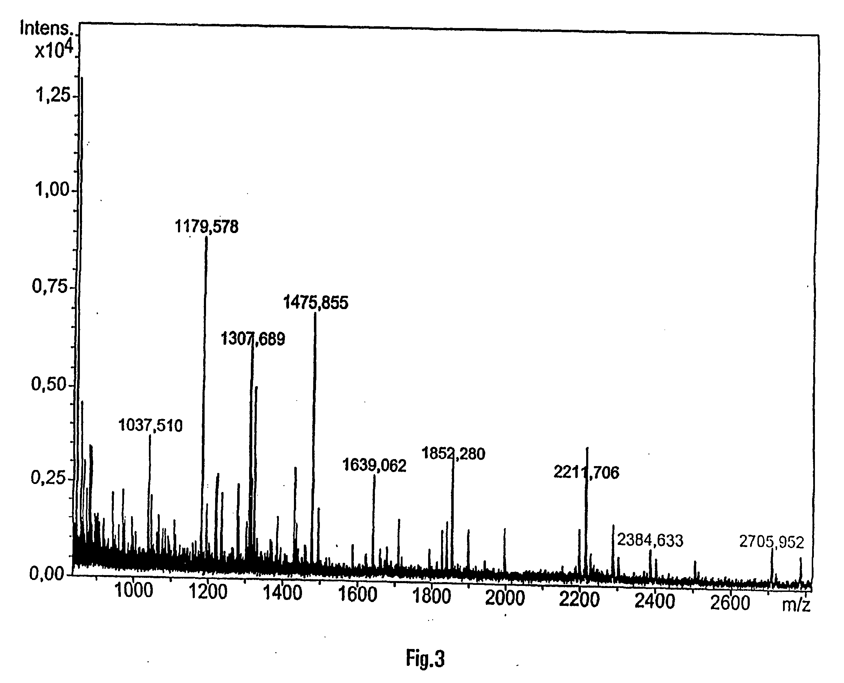

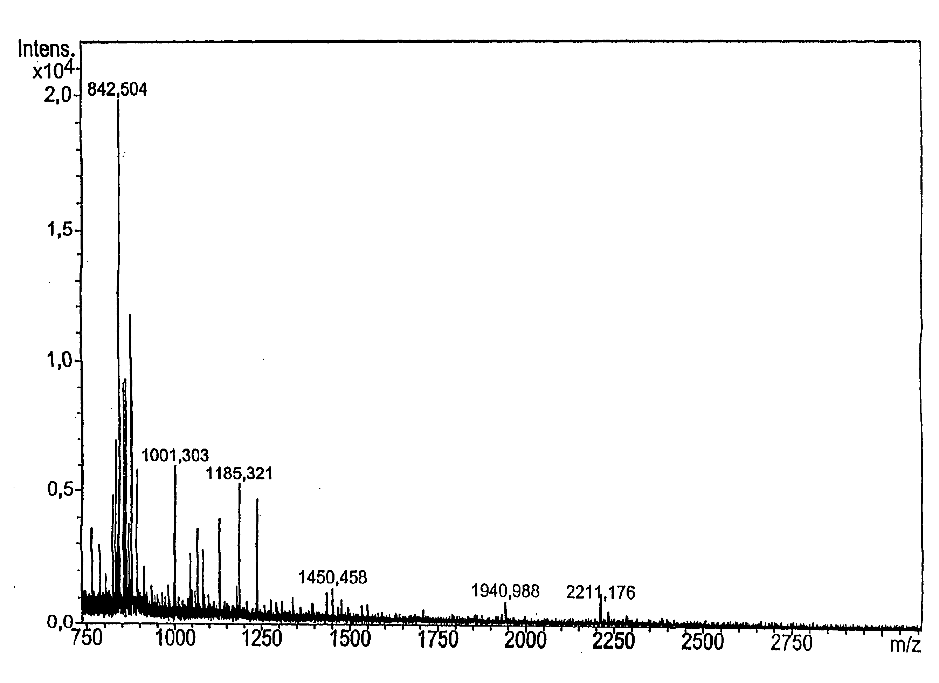

Die für die weitere Analyse vorselektierten Proteinspots wurden aus dem Gel ausgeschnitten, unter Anwendung der Methode, die in Courchesne PL et al., In: Methods in Molecular Biology, Vol. 112:487-513 beschrieben ist, trypsinverdaut und anschließend massenspektroskopisch analysiert und zwar unter Anwendung massenspektrometrischer Untersuchungen, wie sie ebenfalls beschrieben und diskutiert werden in Courchesne PL, a.a.O.. Die bei der Massenspektroskopie (MALDI-MS) der beiden neuen Spots erhaltenen Fragment-Muster (Fig. 3 und 4) wurden dann mit den Datenbanken entnehmbaren Fragment-Mustern von bekannten Proteinen verglichen. Dazu wurden verschiedene Internet-Software-Programme verwendet. Zuerst wurden die durch MALDI-MS ermittelten Peptidmassen mit den theoretischen Peptidmassen aller bekannten Sägetierproteine gematcht. Für die Proteinsuche (in der Owl.7.2.2001-Datenbak mit dem Suchprogramm Protein Prospector) wurden monoisotopische Massen mit Massentoleranzen von 100 ppm (P0) bzw. 200 ppm (L5) zugelassen. Die weitere Suche wurde im Falle des Proteins aus Fig. 1B (P0) mit der NCBinr. Datenbank mit dem Suchprogramm ProFound, im Falle des Proteins aus Fig. 2B mit der Datenbank NCBI (2001/07/20) mit dem Suchprogramm ProFound (Spezies: Mammalia) und einer Massentoleranz von 200 ppm durchgeführt.

Die beiden krankheitstypischen Proteine wurden eindeutig als die an sich bekannten ribosomalen Proteine P0 mit der Aminosäuresequenz gemäß SEQ ID NO:1 und L5 mit der Aminosäuresequenz gemäß SEQ ID NO:2 identifiziert.

### 5. Qualitativer Vergleich zwischen Kontrollen und pathologischen Proben

Indem man gemäß der obigen Prozedur einzelne Affinitatssäulen unter Verwendung von jeweils 10 Seren Gesunder (Kontrollen), 10 Seren von MC-Patienten und 10 Seren von CU-Patienten herstellte und mit dem o.g. Darmextrakt behandelte, wurden die in der folgenden Tabelle gezeigten Ergebnisse erhalten.

**Tabelle**

| Probe | P0 | L5 |
|---|---|---|
| Kontrolle 1 | - | - |
| Kontrolle 2 | - | - |
| Kontrolle 3 | - | - |
| Kontrolle 4 | - | - |
| Kontrolle 5 | - | - |
| Kontrolle 6 | - | - |
| Kontrolle 7 | - | - |
| Kontrolle 8 | - | - |
| Kontrolle 9 | - | - |
| Kontrolle 10 | - | - |
| | | |
| MC-Patient 1 | + | + |
| MC-Patient 2 | + | + |
| MC-Patient 3 | + | + |
| MC-Patient 4 | + | + |
| MC-Patient 5 | + | + |
| MC-Patient 6 | - | + |
| MC-Patient 7 | + | + |
| MC-Patient 8 | + | - |
| MC-Patient 9 | + | + |
| MC-Patient 10 | + | + |
| | | |
| CU-Patient 1 | + | + |
| CU-Patient 2 | - | + |
| CU-Patient 3 | + | + |
| CU-Patient 4 | + | + |
| CU-Patient 5 | - | + |
| CU-Patient 6 | + | + |
| CU-Patient 7 | + | + |
| CU-Patient 8 | + | * |
| CU-Patient 9 | + | - |
| CU-Patient 10 | + | + |

Es ist der Tabelle zu entnehmen, dass bei den meisten MC- und CU-Patienten beide Arten von Antikörpern gleichzeitig zu finden sind, wenigstens eine Art von Antikörpern jedoch bei allen MC bzw. CU-Patienten. Dagegen werden derartige Antikörper in keinem Serum der gesunden Kontrollpersonen gefunden. Bei dem untersuchten relativ beschränkten Kollektiv von Testpersonen war somit die Selektivität 100%. Wenn man beide Antikörper bestimmt und den Nachweis einer Antikörperart als diagnostisch signifikant ansieht, betrug auch die Sensitivität 100%.

### Literaturliste

1. Thomas L., Labor und Diagnose, 5. Auflage, 1998, 824-842.
2. J.-C. Homberg, M. Rizzetto and Deborah Doniach, Ribosomal Antibodies Detected by Immunofluorescence in Systemic Lupus Erythematosus and other Collagenose, Clin.exp.Immunol. (1974) 17, 617-628.
3. J.B.Winfield, Are anti-ribosomal P protein antibodies a type of anti-lymphocyte antibody? Clin.Exp.Immunol 1997, 109, 1-3;
4. Stafford HA, Chen AE, Anderson CJ et al., Anti-ribosomal and 'P-peptide'-specific autoantibodies bind to T lymphocytes, Clin.Exp.Immunol 1997; 109, 12-19.
5. H.-H. Sun, W.-T. Liu, S.-Y. Tang, C.-Y. Tsai, S.-C. Hsieh, T.-H. Wu, S.-H. Han, The expression of acidic ribosomal phosphoproteins on the surface membrane of different tissues in autoimmune and normal mice which are the target molecules for anti-double-stranded DNA antibodies, Immunology 1996, 87, 362-371.
6. David T. Grabowski, Walter A. Deutsch, Dennis Derda and Mark R. Kelley, Drosophila AP3, a presumptive DNA repair protein, is homologous to human ribosomal associated protein P0, Nucleic Acids Resaerch, Vol.19, 4297, 1991
7. Nobuo Kondoh, Toru Wakatsuki, Akihide Ryo, Akiyuki Hada, Tsukasa Aihara, Sankichi Horiuchi, Narihide Goseki, Osamu Matsubara, Kenji Takenaka, Mizue Shichita, Kenji Tanaka, Masahiro Shuda, and Mikio Yamamoto, Indentification and Characterzation of Genes Associated with Human Hepatocellular Carcinogenesis, Cancer Res. 59, 4990-4996, 1999
8. M.A. Rodriguez-Gabriel, M. Remacha, and J.P.G. Ballesta, Phosphorylation of Ribosomal Protein P0 Is Not Essential for Ribosome Function but Can Affect Translation, Biochemistry 1998, 37, 16620-16626
9. G.F.Barnard, R.J. Staniunas, Shideng Bao, Ken-ichi Mafune, G.D. Steele Jr., J.L. Gollan, and Lan Bo Chen, Increased Expression of Human Ribosomal Phosphoprotein P0 Messenger RNA in Hepatocellular Carcinoma and Colon Carcinoma, Cancer Res. 52, 3067-3072, 1992
10. K.-H. Sun, W.-T. Liu, C.-Y. Tsai, S.-J. Tang, S.-H. Han, C.-L. Yu, Anti-dsDNA antibodies cross-react with ribosomal P proteins expressed on the surface of glomerular mesangial cells to exert a cytostatic effect, Immunology 1995, 85, 262-269
11. I. Ferrari, M.J. Levin, G. Wallukat, R. Elies, D. Lebesgue, P. Chiale, M. Elizari, M. Rosenbaum, and J. Hoebeke, Molecular Mimicry between the Immunodominant Ribosomal Protein P0 of Trypanosoma cruzi and a Functional Epitope on the Human β₁-Adrenergic Receptor, J.Exp.Med., Vol. 182, 1995, 59-65
12. S. Chatterjee, S. Singh, R. Sohoni, V. Kattige, Ch. Deshpande, Sh. Chiplunkar, N. Kumar, Sh. Sharma, Characterization of domains of the phosphoriboprotein P0 of Plasmodium falciparum, Mol.Biochem.Parasitol. 107 (2000) 143-154
13. S. Chatterjee, S. Singh, R. Sohoni, N.J. Singh, A. Vaidya, C. Long, and Sh. Sharma, Antibodies against Ribosomal Phosphoprotein P0 of Plasmodium falciparum Protect Mice against Challenge with Plasmodium yoelli, Infect.Immun., Vol.68, 2000, 4312-4318
14. A. Goswami, S. Singh, V.D. Redkar, and Sh. Sharma, Characterization of P0, a Ribosmoal Phosphoprotein of Plasmodium falciparum, J.Biol.Chem. Vol.272, 12138-12143, 1997
15. N. Fabien et al., Autoantibodies Directed Against the Ribosomal P Proteins are not only Directed Against a Common Epitope of the P0, P1 and P2 Proteins, J.Autoimmun. (1999) 13, 103-110
16. B. Liliensiek at al., Identification of Four Genes in Endothelial Cells Whose Expression Is Affected by Tumor Cells and Host Immune Status - A Study in Ex Vivo-Isolated Endothelial Cells, Blood, Vol.92 (9), 1998, 3394-3404
17. R. Szyszka, G. Bou, J.P.G. Ballesta, RAP Kinase, a new enzyme phosphorylating the acidic P proteins from Saccharomyces cerevisiae, Biochim.Biophys.Acta 1293, (1996), 213-221
18. O. Rosorius et al., Human Ribosomal Protein L5 Contains Defined Nuclear Localization and Export Signals, J.Biol.Chem., Vol.275, 12061-12068, 2000
19. K.Hirano et al., Interaction of the Ribosomal Protein, L5, with Protein Phosphatase Type 1, J.Biol.Chem. Vol.270, 19786-19790, 1995
20. W.M. Michael and G. Dreyfuss, Distinct Domains in Ribosomal Protein L5 Mediate 5 S rRNA Binding and Nuclear Localization, J.Biol.Chem. Vol.271, 11571-11574, 1996
21. M. Claußen, F. Rudt, and T. Pieler, Functional Modules in Ribosomal Protein L5 for Ribonucleoprotein Complex Formation and Nucleocytoplasmic Transport, J.Biol.Chem. Vol.274, 33951-33958, 1999
22. A.Giualis et al., Anti-5S RNA/protein (RNP) antibody levels correlate with disease activity in a patient with systemic lupus erythematosus (SLE) nephritis, Clin.Exp.Immunol. 1994, 95, 385-389
23. V. Marechal et al., The Ribosomal L5 Protein Is Associated with mdm-2 and mdm-2-p53 Complexes, Mol.Cell.Biol. Vol. 14, 7414-7420, 1994
24. O.Spirina et al., Heart-specific splice-variant of a human mitochondrial ribosomal protein (mRNA processing; tissue specific splicing), Gene 261 (2000) 229-234
25. J.-M. Kim et al., Interaction of the β Subunit of Casein Kinase II with the Ribosomal Protein L5, BBRC 226, 180-186 (1996)
26. J.-M. Frigerio, J.-C. Dagorn, J.L. Iovanna, Cloning, sequencing and expression of the L5, L21, L27a, L28, S5, S9, S10 and S29 human ribosomal protein mRNAs, Biochim.Biophys.Acta 1262 (1995) 64-68
27. B. Guerra, O.-G. Issinger, p53 and the ribosomal protein L5 participate in high molecular mass complex formation with protein kinase CK2 in murine teratocarcinoma cell line F9 after serum stimulation and cis-platin treatment, FEBS Letters 434 (1998) 115-120

## Patentansprüche

1. Verfahren zur Diagnose, zur Frühdiagnose, zur Differentialdiagnose, zur Beurteilung der Schwere und zur therapiebegleitenden Verlaufskontrolle und Verlaufsprognose von chronisch entzündlichen Darmerkrankungen, **dadurch gekennzeichnet, dass** man im Serum, Plasma, Gewebeproben und/oder Stuhl eines Patienten, der an einer chronisch entzündlichen Darmerkrankung leidet, oder bei dem der Verdacht auf eine derartige Erkrankung besteht, das Vorhandensein und/oder die Menge von einem oder mehreren Antikörpern bestimmt, die an ribosomale Proteine binden.

2. Verfahren nach Anspruch 1, bei dem die chronisch entzündliche Darmerkrankung ausgewählt ist aus Morbus Crohn und Colitis ulcerosa oder ihrer als Colitis indeterminata bezeichneten Mischform.

3. Verfahren nach Anspruch 1, **dadurch gekennzeichnet, dass** man wenigstens einen Antikörper bestimmt, der aus der Gruppe von Antikörpern ausgewählt ist, die an das ribosomale Protein P0 oder an das ribosomale Protein L5 binden.

4. Verfahren nach Anspruch 1, 2 oder 3, **dadurch gekennzeichnet, dass** man den wenigstens einen Antikörper mit Hilfe eines Immunoassays bestimmt, bei dem das jeweilige ribosomale Protein als Antigen zur Bindung und/oder Markierung der zu bestimmenden Antikörper verwendet wird.

5. Verfahren nach Anspruch 4, **dadurch gekennzeichnet, dass** das ribosomale Protein ein humanes oder tierisches Protein ist, das aus natürlichen Quellen angereichert oder isoliert wurde oder auf gentechnologischem Wege (rekombinant) erzeugt wurde.

6. Verwendung des ribosomalen Proteins P0 und/oder des ribosomalen Proteins L5 zur Herstellung eines Arzneimittels zur Bindung oder Blockierung von Antikörpern, die an das ribosomale Protein P0 binden, und/oder von Antikörpern, die an das ribosomale Protein L5 binden, für die Therapie des Morbus Crohn und/oder der Colitis ulcerosa.

7. Verwendung des ribosomalen Proteins P0 und/oder des ribosomalen Proteins L5 zur Herstellung eines Arzneimittels zur Hemmung der Darmentzündung und assoziierter Entzündungen bei Morbus Crohn und/oder der Colitis ulcerosa durch Blockierung antigenpräsentierender Zellen oder zur Erzeugung einer T-Zellanergie.

8. Verwendung des ribosomalen Proteins P0 und/oder des ribosomalen Proteins L5 zur Herstellung eines Affinitätsmaterials zur extrakorporalen Entfernung von Antikörpern, die an das ribosomale Protein P0 binden, und/oder von Antikörpern, die an das ribosomale Protein L5 binden, aus dem Blutkreislauf eines Patienten.

## Claims

1. Method for diagnosis, early diagnosis, differential diagnosis, assessment of the severity and therapy-accompanying monitoring and prognosis of chronic inflammatory bowel diseases, **characterized in that** the presence and/or the amount of one or more antibodies which bind to ribosomal proteins are determined in the serum, plasma, tissue samples and/or stool of a patient who is suffering from a chronic inflammatory bowel disease or in whom such a disease is suspected.

2. Method according to Claim 1, in which the chronic inflammatory bowel disease is selected from Crohn's disease and ulcerative colitis or their mixed form known as Colitis indeterminata.

3. Method according to Claim 1, **characterized in that** at least one antibody which is selected from the group of antibodies which bind to the ribosomal protein P0 or to the ribosomal protein L5 is determined.

4. Method according to Claim 1, 2 or 3, **characterized in that** the at least one antibody is determined with the aid of an immunoassay in which the respective ribosomal protein is used as antigen for binding and/or labeling the antibodies to be determined.

5. Method according to Claim 4, **characterized in that** the ribosomal protein is a human or animal protein which was enriched or isolated from natural sources or was (recombinantly) produced by a genetic engineering method.

6. Use of the ribosomal protein P0 and/or of the ribosomal protein L5 for the preparation of a medical agent for the binding or blocking of antibodies which bind to the ribosomal protein P0 and/or of antibodies which bind to the ribosomal protein L5, for the therapy of Crohn's disease and/or of ulcerative colitis.

7. Use of the ribosomal protein P0 and/or of the ribosomal protein L5 for the preparation of a medical agent for inhibiting the intestinal inflammation and associated inflammations in Crohn' s disease and/or ulcerative colitis by blocking antigen-presenting cells or for producing a T-cell anergy.

8. Use of the ribosomal protein P0 and/or of the ribosomal protein L5 for the preparation of an affinity material for the extracorporeal removal of antibodies which bind to the ribosomal protein P0 and/or of antibodies which bind to the ribosomal protein L5 from the blood circulation of a patient.

## Revendications

1. Procédé pour le diagnostic, le diagnostic précoce, le diagnostic différentiel, l'appréciation de la sévérité et le suivi et le pronostic de l'évolution accompagnant la thérapie, de maladies intestinales inflammatoires chroniques, **caractérisé en ce que** l'on détermine dans le sérum, le plasma, des échantillons de tissu et/ou les selles d'un patient qui souffre d'une maladie intestinale inflammatoire chronique, ou chez qui on soupçonne une telle maladie, la présence et/ou la quantité d'un ou plusieurs anticorps qui se lient à des protéines ribosomales.

2. Procédé selon la revendication 1, dans lequel la maladie intestinale inflammatoire chronique est choisie parmi la maladie de Crohn et Colitis ulcerosa, ou leur forme mixte désignée par Colitis indeterminata.

3. Procédé selon la revendication 1, **caractérisé en ce que** l'on détermine au moins un anticorps qui est choisi dans le groupe des anticorps qui se lient à la protéine ribosomale PO ou à la protéine ribosomale L5.

4. Procédé selon la revendication 1, 2 ou 3, **caractérisé en ce que** l'on détermine au moins un anticorps à l'aide d'un test immunologique dans lequel la protéine ribosomale en cause est utilisée comme antigène pour une liaison à et/ou un marquage d'un anticorps déterminé.

5. Procédé selon la revendication 4, **caractérisé en ce que** la protéine ribosomale est une protéine humaine ou animale, qui a été concentrée ou isolée à partir de sources naturelles, ou a été produite par voie d'une technique génétique (recombinante).

6. Utilisation de la protéine ribosomale P0 et/ou de la protéine ribosomale L5 pour produire un agent thérapeutique de liaison à, ou de blocage d'anticorps, qui se lient à la protéine ribosomale P0 et/ou d'anticorps qui se lient à la protéine ribosomale L5, pour la thérapie de la maladie de Crohn et/ou de Colitis ulcerosa.

7. Utilisation de la protéine ribosomale P0 et/ou de la protéine ribosomale L5 pour la production d'un agent thérapeutique pour stopper l'inflammation de l'intestin et des inflammations associées dans la maladie de Crohn et/ou Colitis ulcerosa, par blocage de cellules présentant des antigènes, ou pour produire une anergie des cellules T.

8. Utilisation de la protéine ribosomale P0 et/ou de la protéine ribosomale L5 pour la production d'un matériau d'affinité pour éliminer de manière extracorporelle de la circulation sanguine d'un patient, des anticorps qui se lient à la protéine ribosomale P0 et/ou des anticorps qui se lient à la protéine ribosomale L5.


### SEQUENCE LISTING

<110> B.R.A.H.M.S Aktiengesellschaft
<120> Verfahren und Mittel für die Diagnose und Therapie von chronischen entzündlichen Darmerkrankungen
<130> 3618 PCT
<140>
   <141>
<150> 10147991.3 DE
   <151> 2001-09-28
<160> 2
<170> PatentIn Ver. 2.1
<210> 1
   <211> 296
   <212> PRT
   <213> HOMO SAPIENS
<400> 1
   Gly Phe Val Lys Val Val Lys Asn Lys Ala Tyr Phe Lys Arg Tyr Gln
     1               5                  10                  15
   Val Lys Phe Arg Arg Arg Arg Glu Gly Lys Thr Asp Tyr Tyr Ala Arg
                20                  25                  30
   Lys Arg Leu Val Ile Gln Asp Lys Asn Lys Tyr Asn Thr Pro Lys Tyr
            35                  40                  45
   Arg Met Ile Val Arg Val Thr Asn Arg Asp Ile Ile Cys Gln Ile Ala
        50                  55                  60
   Tyr Ala Arg Ile Glu Gly Asp Met Ile Val Cys Ala Arg Tyr Ala His
    65                  70                  75                  80
   Glu Leu Pro Lys Tyr Gly Val Lys Val Gly Leu Thr Asn Tyr Ala Ala
                    85                  90                  95
   Ala Tyr Cys Thr Gly Leu Leu Leu Ala Arg Arg Leu Leu Asn Arg Phe
   100 105 110
   Gly Met Asp Lys Ile Tyr Glu Gly Gln Val Glu Val Thr Gly Asp Glu
   115 120 125
   Tyr Asn Val Glu Ser Ile Asp Gly Gln Pro Gly Ala Phe Thr Cys Tyr
   130 135 140
   Leu Asp Ala Gly Leu Ala Arg Thr Thr Thr Gly Asn Lys Val Phe Gly
   145 150 155 160
   Ala Leu Lys Gly Ala Val Asp Gly Gly Leu Ser Ile Pro His Ser Thr
   165 170 175
   Lys Arg Phe Pro Gly Tyr Asp Ser Glu Ser Lys Glu Phe Asn Ala Glu
   180 185 190
   Val His Arg Lys His Ile Met Gly Gln Asn Val Ala Asp Tyr Met Arg
   195 200 205
   Tyr Leu Met Glu Glu Asp Glu Asp Ala Tyr Lys Lys Gln Phe Ser Gln
   210 215 220
   Tyr Ile Lys Asn Ser Val Thr Pro Asp Met Met Glu Glu Met Tyr Lys
   225 230 235 240
   Lys Ala His Ala Ala Ile Arg Glu Asn Pro Val Tyr Glu Lys Lys Pro
   245 250 255
   Lys Lys Glu Val Lys Lys Lys Arg Trp Asn Arg Pro Lys Met Ser Leu
   260 265 270
   Ala Gln Lys Lys Asp Arg Val Ala Gln Lys Lys Ala Ser Phe Leu Arg
   275 280 285
   Ala Gln Glu Arg Ala Ala Glu Ser
   290 295
<210> 2
   <211> 317
   <212> PRT
   <213> HOMO SAPIENS
<400> 2
   Met Pro Arg Glu Asp Arg Ala Thr Trp Lys Ser Asn Tyr Phe Leu Lys
    1               5                  10                  15
   Ile Ile Gln Leu Leu Asp Asp Tyr Pro Lys Cys Phe Ile Val Gly Ala
               20                  25                  30
   Asp Asn Val Gly Ser Lys Gln Met Gln Gln Ile Arg Met Ser Leu Arg
           35                  40                  45
   Gly Lys Ala Val Val Leu Met Gly Lys Asn Thr Met Met Arg Lys Ala
       50                  55                  60
   Ile Arg Gly His Leu Glu Asn Asn Pro Ala Leu Glu Lys Leu Leu Pro
   65                  70                  75                  80
   His Ile Arg Gly Asn Val Gly Phe Val Phe Thr Lys Glu Asp Leu Thr
                   85                  90                  95
   Glu Ile Arg Asp Met Leu Leu Ala Asn Lys Val Pro Ala Ala Ala Arg
               100                 105                 110
   Ala Gly Ala Ile Ala Pro Cys Glu Val Thr Val Pro Ala Gln Asn Thr
           115                 120                 125
   Gly Leu Gly Pro Glu Lys Thr Ser Phe Phe Gln Ala Leu Gly Ile Thr
       130                 135                 140
   Thr Lys Ile Ser Arg Gly Thr Ile Glu Ile Leu Ser Asp Val Gln Leu
   145                 150                 155                 160
   Ile Lys Thr Gly Asp Lys Val Gly Ala Ser Glu Ala Thr Leu Leu Asn
                   165                 170                 175
   Met Leu Asn Ile Ser Pro Phe Ser Phe Gly Leu Val Ile Gln Gln Val
               180                 185                 190
   Phe Asp Asn Gly Ser Ile Tyr Asn Pro Glu Val Leu Asp Ile Thr Glu
           195                 200                 205
   Glu Thr Leu His Ser Arg Phe Leu Glu Gly Val Arg Asn Val Ala Ser
       210                 215                 220
   Val Cys Leu Gln Ile Gly Tyr Pro Thr Val Ala Ser Val Pro His Ser
   225                 230                 235                 240
   Ile Ile Asn Gly Tyr Lys Arg Val Leu Ala Leu Ser Val Glu Thr Asp
                   245                 250                 255
   Tyr Thr Phe Pro Leu Ala Glu Lys Val Lys Ala Phe Leu Ala Asp Pro
               260                 265                 270
   Ser Ala Phe Val Ala Ala Ala Pro Val Ala Ala Ala Thr Thr Ala Ala
           275                 280                 285
   Pro Ala Ala Ala Ala Ala Pro Ala Lys Val Glu Ala Lys Glu Glu Ser
       290                 295                 300
   Glu Glu Ser Asp Glu Asp Met Gly Phe Gly Leu Phe Asp
   305                 310                 315